# EUROPEAN PATENT APPLICATION

(11) **EP 0 926 499 A1**
(43) Date of publication of application: **30.06.1999**
(21) Application number: 97933000.8
(22) Date of filing: 24.07.1997
(51) Int. Cl.: G01N 33/68, G01N 33/53, G01N 33/531

(54) **METHOD FOR PREVENTING ADSORPTION OF LACTOFERRIN**

(30) Priority: 30.07.1996 JP 20006796
(71) Applicant: SHIONOGI & CO., LTD., Osaka-shi, Osaka 541-0045 (JP)
(72) Inventor: FUKUNAGA, Takahiro, Osaka 533 (JP)
(74) Representative: VOSSIUS & PARTNER
(86) International application number: JP9702558
(87) International publication number: WO9804922

(57) **Abstract**

The present invention relates to a method for preventing the adsorption of lactoferrin to a storage container or a reaction container characterized by regulating the pH value of a lactoferrin-containing solution in the container not to exceed 4, or regulating the salt concentration of the solution, for example, at least 1.0 M in the case of NaCl or at least 0.5 M in the case of KCl; a lactoferrin containing solution which has a pH value of at most 4, a sodium chloride concentration of at least 1.0 M or a potassium chloride concentration of at least 0.5 M; and a kit for assaying lactoferrin which comprises the above-mentioned solution as a constituting reagent.

## Description

### Technical Field

This invention relates to a method for preparing a solution containing lactoferrin for exact determination of quantity of lactoferrin. More specifically, it relates to a method for preventing the adsorption of lactoferrin to determine exact quantity of lactoferrin.

### Background Art

Lactoferrin is a iron-binding protein mainly synthesized by granulocytes. Lactoferrin has strong iron-binding ability, and is one of biological defence factors having a strong bacteriostatic activity against iron requirement pathogenic bacteria. Through this activity, lactoferrin is usually abundant in exocrine secretions such as milk and saliva to prevent infections by pathogenic bacteria. However, if an inflammation occurred in a living body by a certain reason, a large amount of lactoferrin would be secreted because granulocytes wander toward and activate the inflammation part. Therefore, lactoferrin is important as an inflammation marker and an assay kit for lactoferrin has already been on the market, for example, Lactof-EIA (BIOXYTECH, France).

Usually, immunoassay methods, which is good at specificity, such as the ELISA method, the RIA method and Rapid Test detecting reagents are used for determining quantity of lactoferrin. It is indispensable to use lactoferrin as a standard substance or a labeled substance in immunoassay methods. The use of lactoferrin, however, involves a problem that lactoferrin is adsorbed to walls of storage containers and reaction containers due to its unique property. Apparent quantity of lactoferrin in a standard solution would decrease to less than indicated exact quantity during storage because the lactoferrin as a standard substance is adsorbed to a wall of a container in a standard solution previously prepared. On the other hand, apparent quantity of lactoferrin in specimens or samples would decrease to less than exact quantity, because the lactoferrin is adsorbed to a wall of a reaction container, which would lead to errors of determination. Consequently, if the fact of the adsorption of lactoferrin to walls of containers was ignored, many types of problems would happen.

A fact that lactoferrin is adsorbed to walls of storage containers and reaction containers which consists of usual materials such as glass, plastic, and the like and a method for preventing the adsorption of lactoferrin have not been known.

The present inventor confirmed that this adsorption of lactoferrin to container walls could not be prevented by conventional methods for preventing the adsorption, that is, addition of proteins such as bovine serum albumin (BSA), casein, etc.

The present invention relates to a method for preventing the adsorption of lactoferrin to determine exact quantity of lactoferrin and it further provides a method for certain detecting inflammatory diseases or diseases such as cancer to cause incidental inflammations.

### Disclosure of Invention

The invention relates to a method for preventing the adsorption of lactoferrin to a storage container or a reaction container characterized by regulating the pH value of a lactoferrin-containing solution in the container so as not to exceed 4, or regulating the salt concentration of the solution to, for example, at least 1.0 M in the case of sodium chloride or at least 0.5 M in the case of potassium chloride. Additionally, it also relates to a lactoferrin-containing solution which has a pH value of at most 4, a sodium chloride concentration of at least 1.0 M or a potassium chloride concentration of at least 0.5 M and a kit for assaying lactoferrin which comprises the above-mentioned solution as a constituting reagent.

In this invention, an acidic buffer solution can be used to regulate the pH value of a lactoferrin-containing solution so as not to exceed 4. As an acidic buffer solution, a citrate buffer solution or an acetate buffer solution is preferred. A glycine buffer solution, a hydrochloride buffer solution etc. can be also used. Preferably, the pH is regulated by using 10 mM citrate buffer solution or 10 mM acetate buffer solution.

Regulating a salt concentration of the lactoferrin-containing solution is also the feature of this invention. As a salt, sodium chloride (NaCl), potassium chloride (KCl) are given for examples. K₃Fe(CN)₆ etc. can be also used.

The method of this invention can be applied to any types of immunoassay methods. It can be used in usually used assay methods such as an enzyme immunoassay, a radio immunoassay, a fluorescent immunoassay, a luminescent immunoassay, a latex agglutination method etc.

Usually, a solution which has a pH value of at most 4, a sodium chloride concentration of at least 1.0 M or a potassium chloride concentration of at least 0.5 M is kept in a vial of a lactoferrin-assaying kit. This solution is used as a diluent in creating a standard curve and as a diluent for specimens or samples to prepare the lactoferrin-containing solution.

### Brief Description of Drawings

Figure 1 is a graph showing the adsorption of lactoferrin to glass and polypropylene. Micro tube, Assist tube and Falcon tube in the figure are made of polypropylene.
Figure 2 is a graph showing an effect of pH value changes in preventing the adsorption of lactoferrin.
Figure 3 is a graph showing an effect of NaCl concentration changes in preventing the adsorption of lactoferrin.
Figure 4 is a graph showing an effect of KCl concentration changes in preventing the adsorption of lactoferrin.

### Best Mode for Carrying Out the Invention

This invention is explained in more detail by the following reference example and examples, but it is not to be construed to limit the scope of this invention. The lactoferrin determination in the following reference example and the examples were performed as follows, unless otherwise described:

### 1 ) Immobilization of Antibody to Microplate

To each well of microplates (NUNC, Denmark), 250 µl of 0.01 M PBS buffer solution containing 10 µg/ml of anti-human lactoferrin antibody (DAKOPATTS, Denmark) was poured and left at 4 °C for 12 hr to prepare antibody-immobilized plates.

### 2 ) Preparation of Enzyme-Labeled Antibody

Horseradish peroxidase (HRP) was bound to an Fab' fragment of an anti-human lactoferrin antibody at a ratio of one to one. The HRP-labeled anti-human lactoferrin antibody thus obtained was prepared to be 30 ng/ml for use.

### 3 ) Determination of Quantity of Lactoferrin

To each well of the microplates prepared in step 1), 0.5 M Tris-hydrochloride buffer solution (pH 7.5) containing 0.1 % bovine serum albumin and 1.25 M NaCl was dispensed'. Then, 50 µl of standard lactoferrin (Cappel, Sweden) diluted to 20 to 720 ng/ml by the same buffer solution was added and mixed, followed by the reaction at 25 °C for 1 hr. After washing three times with 0.01 M Tris-hydrochloride buffer solution (pH 7.0) containing 0.05 % Tween 20, the HRP-labeled anti-human lactoferrin antibody prepared in step 2) were added at 100µl to the each well. After mixed and reacted at 25 °C for 1 hr, the same wash as above was carried out. TMB coloring reagent (BioRad, USA) was added at 100 µl, mixed, and reacted at 25 °C for 15 min. The reaction was terminated by 1 M phosphoric acid. Coloring of the each well was measured by a colorimeter for microplate at wave length of 450 nm. Quantity of lactoferrin in each specimen or sample was calculated from the standard curve.

### Reference example

### Confirmation of Adsorption of Lactoferrin

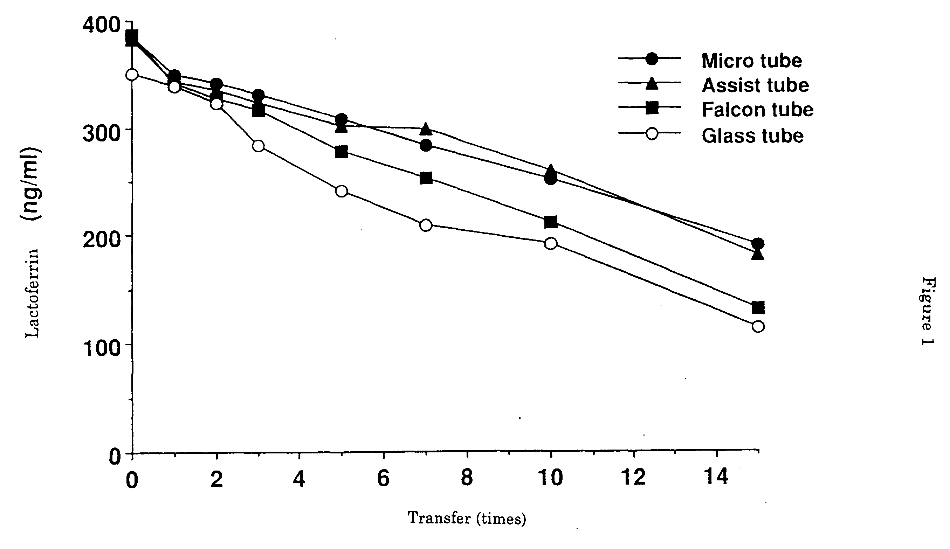

To confirm the adsorption of lactoferrin, materials usually used for clinical laboratory tests such as glass and polypropylene (Micro tube, Assist tube, and Falcon tube in the Figure) were used for this test. To test tubes (10 ml volume) made of each material, 3 ml of 0.05 M PBS (phosphate buffered saline, pH 7.4) and 1.5 µg of standard lactoferrin (CAPPEL) were : added. After stirring it enough by a mixer, 2.8 ml of the solution was transferred to another test tube made of the same material. The remained 0.2 ml solution in each test tube was used as a sample to determine the quantity of lactoferrin. This operation was repeated 10 times in the same way, and the quantity of lactoferrin was determined each time. The obtained results are shown in Figure 1 by the average of two tests.

If the determined lactoferrin concentrations were getting lower as the repeating times were getting more, this means that lactoferrin has been adsorbed to the material. Figure 1 shows that lactoferrin has strongly been adsorbed to both the materials and a careful treatment is required from sample collection to determination.

### Example 1

### Prevention of Adsorption of Lactoferrin

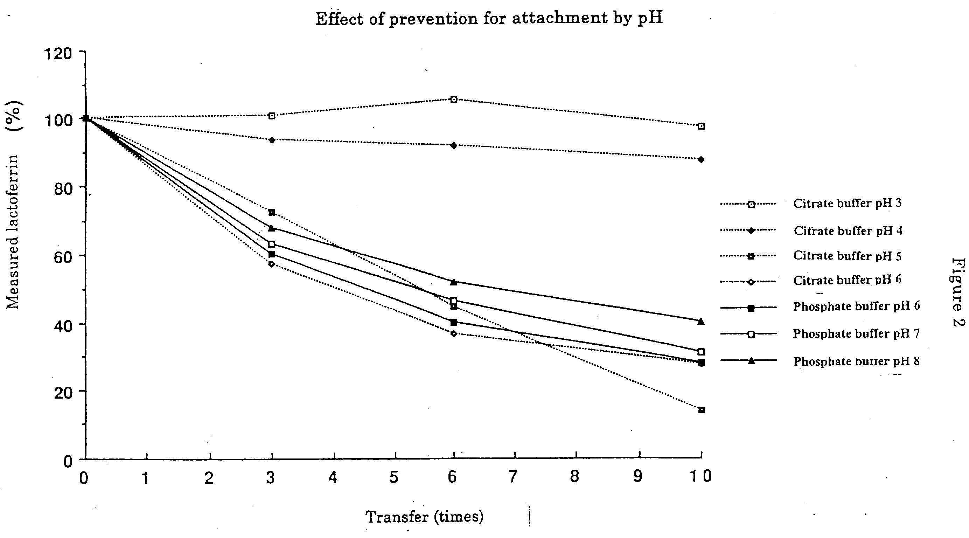

Prepared were 360 ng/ml solutions of standard lactoferrin using the solutions whose pH values were adjusted to 3, 4, 5 and 6 by mixing 10 mM citric acid and 10 mM sodium citrate. Using tubes made of glass, the tests were repeated in the same way as described in the reference example. The obtained results are shown in Figure 2 by the average of two tests. For reference, the same test was performed by using the solution of which pH value was adjusted to 6 - 8 by phosphoric acid.

### Example 2

### Prevention of Adsorption of Lactoferrin

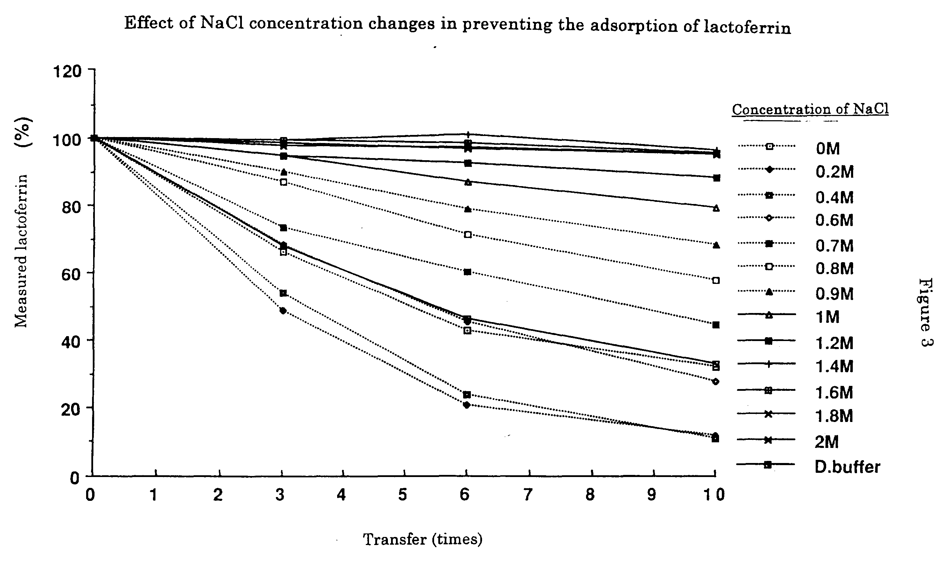

Using the solutions of 0 - 2 M NaCl prepared by mixing 0 M NaCl and 2 M NaCl, 360 ng/ml solutions of standard lactoferrin were prepared. Using tubes made of glass, the tests were repeated in the same way as described in the reference example. The obtained results are shown in Figure 3 by the average of two tests.

### Example 3

### Prevention of Adsorption of Lactoferrin

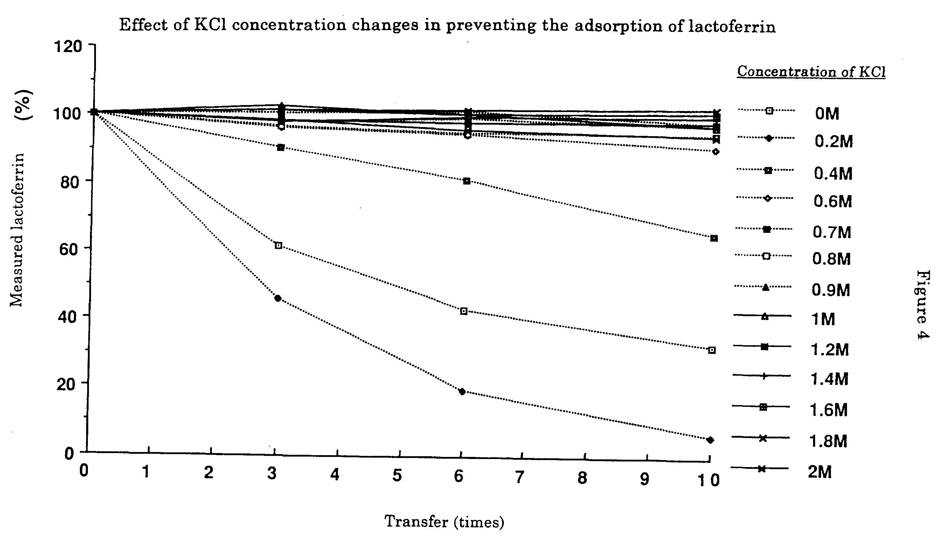

Using the solutions of 0 - 2 M KCl prepared by mixing 0 M KCl and 2 M KCl, 360 ng/ml solutions of standard lactoferrin were prepared. Using tubes made of glass, the tests were repeated in the same way as described in the reference example. The obtained results are shown in Figure 4 by the average of two tests.

### Conclusion

Figures 2 to 4 show that declining a pH value or raising a salt concentration is effective for preventing the adsorption of lactoferrin. Determination error is between ± 10 % to 20 % at the ELISA. Therefore, the Figures 2, 3 and 4 show that the lactoferrin-containing solution should be kept at most pH 4, at least 1.0 M NaCl, or at least 0.5 M KCl to prevent the adsorption of lactoferrin.

## Claims

1. A method for preventing the adsorption of lactoferrin to a storage container or a reaction container which comprises regulating the pH value of a lactoferrin-containing solution in the container not to exceed 4.

2. The method as claimed in claim 1, in which an acidic buffer solution able to keep the pH value from 3 to 4 is used.

3. The method as claimed in claim 2, in which the buffer solution is a citrate buffer solution, an acetate buffer solution or a glycine buffer solution.

4. A method for preventing the adsorption of lactoferrin to a storage container or a reaction container which comprises regulating the salt concentration of a lactoferrin-containing solution in the container.

5. The method as claimed in claim 4, in which the salt concentration is at least 1.0 M in the case of sodium chloride or at least 0.5 M in the case of potassium chloride.

6. A lactoferrin-containing solution which has a pH value of at most 4, a sodium chloride concentration of at least 1.0 M or a potassium chloride concentration of at least 0.5 M.

7. A kit for assaying lactoferrin which comprises the solution as claimed in claim 6 as a constituting reagent.

8. A kit for assaying lactoferrin which contains a solution having a pH value of at most 4, a sodium chloride concentration of at least 1.0 M or a potassium chloride concentration at least 0.5 M.
